# EUROPÄISCHE PATENTSCHRIFT

(11) **EP 3 708 112 B1**
(45) Veröffentlichungstag und Bekanntmachung des Hinweises auf die Patenterteilung: **17.07.2024**
(21) Anmeldenummer: 19215633.9
(22) Anmeldetag: 12.12.2019
(51) Int. Cl.: A61C 5/90

(54) **FOLIENSPANNELEMENT**
FILM TENSIONING ELEMENT
ÉLÉMENT DE SERRAGE DE FEUILLE

(30) Priorität: 13.12.2018 EP 18212441; 12.12.2018 EP 18212089
(43) Veröffentlichungstag der Anmeldung: 16.09.2020
(73) Patentinhaber: Ivoclar Vivadent AG, 9494 Schaan (LI)
(72) Erfinder: Schmid, André, 9452 Hinterforst (CH); Enggist, Lukas, 7320 Sargans (CH); Müller, Frank, 6800 Feldkirch (AT); Atilim, Eser, 88131 Bodolz (DE)
(74) Vertreter: Baldus, Oliver

(56) Entgegenhaltungen:
- EP-A1- 1 709 935
- DE-A1- 3 329 919
- US-A1- 2004 049 099

## Beschreibung

Die Erfindung betrifft ein Folienspannelement, gemäß dem Oberbegriff von Anspruch 1, 11 bzw. 13.

Derartige Folienspannelemente sind seid langem bekannt. Beispielhaft sei insofern auf die WO 03/51185 A1 verwiesen. Mit solchen Folienspannelementen soll ein freier Zugang zum Mund des Patienten gewährleistet sein. Hierzu ist eine oval- oder kreisringförmig angeordnete und sich zwischen zwei Ringen, dem sogenannten Lippenring und dem sogenannten Vestibulärring, erstreckende Folie vorgesehen. Die Folie kann auch auf einem der Ringe verschieblich gelagert sein.

Sie ist jedenfalls elastisch und passt sich insofern in dem Verlauf der Lippen des Patienten an und deckt die Lippen zugleich ab.

Sowohl der Lippenring als auch der Vestibulärring sind im Vergleich mit dem Folienelement vergleichsweise steif. Sie spannen daher das Folienelement zwischen sich auf, wobei das Folienelement den Mund des Patienten gleichsam aufweitet.

Die anatomischen Gegebenheiten des Mundraums und insbesondere des Vestibulums und des Lippenbereichs sind zwischen verschiedenen Patienten in aller Regel deutlich unterschiedlich. Dem soll durch die Elastizität des Folienelements bei den bekannten Lösungen Rechnung betragen werden. Die Folie erstreckt sich dabei von dem Vestibulum des Patienten über die Einschnürung an den Lippen hinweg bis nach außen, wobei ein vergleichsweise großer Lippenring die auftretenden Kräfte besser verteilen soll.

Insbesondere im Mundinnenraum, also im Bereich des Vestibulärrings, wird die eingeleitete Spannung vom Patienten häufig als unangenehm empfunden, da sich der Vestibulärring häufig am Kieferknochen des Patienten abstützt.

Aus der EP 3 160 381 A1 ist es daher bekannt geworden, ein Vorspannkorsett zu erzeugen, das dem Frenulum Rechnung trägt und so die eingeleiteten Spannung mindert.

Dies stellt eine erhebliche Verbesserung gegenüber der ursprünglichen Lösung dar, ist aber im Grunde noch weiter verbesserungswürdig.

Aus der DE3329919 ist ein Lippenschutz bekannt zum Einsatz während einer ärztlichen, vorzugsweise chirurgischen Behandlung der Zähne und/oder des Mund- oder Rachenraumes.

Ein weiteres Problem der bislang verwendeten Folienspannelemente ist der von Patient zu Patient recht unterschiedliche Druck, der auf die Lippen ausgeübt wird.

Dieser hängt einerseits von der Größe der beiden Ringe im Verhältnis zu der Mundöffnung des Patienten ab, andererseits natürlich von den verwendeten Materialien.

Ein zu großer Druck insbesondere auf die Kieferknochen und die Frenula wird als unangenehm empfunden. Andererseits wird die Aufspannfunktion des Folienspannelements wesentlich von der radial nach außen wirkenden Kraft getragen. Um eine Anpassung an unterschiedliche Anatomien unterschiedlicher Patienten vorzunehmen, werden daher Folienspannelemente typischerweise in mehreren Größen vorrätig gehalten.

Da die Folie extrem elastisch sein muss, wird häufig ein hierfür geeignetes Material verwendet, beispielsweise ein Elastamer oder Weichsilikon.

Demgegenüber liegt der Erfindung die Aufgabe zu Grunde, ein Folienspannelement gemäß dem Oberbegriff von Anspruch 1, 11 bzw. 13 zu schaffen, das eine bessere Akzeptanz erlaubt.

Diese Aufgabe wird erfindungsgemäß durch Anspruch 1, 11 bzw. 13 gelöst. Vorteilhafte Weiterbildungen ergeben sich aus den Unteransprüchen.

Das erfindungsgemäße Folienspannelement zeichnet sich erfindungsgemäß dadurch aus, dass durch ein besonderes Elastikband, das sich ringförmig um die Folie oder entlang dieser erstreckt, und zwar im Bereich zwischen den beiden Ringen, die Folie dort radial einwärts gezogen wird. Zwar müsste diese Elastikband der Abhaltefunktion des Folienspannelements an sich entgegenwirken. Im eingesetzten Zustand kommt das Elastikband jedoch entweder in der Übergangszone zwischen der Außenseite und der Innenseite der Lippe zu liegen oder auf der Innenseite der Lippe.

Dadurch bewirkt es eine Zugkraft auf den Vestibulärring und zieht diesen von dem Alveolarkamm und von dem Lippenbändern weg. Insofern liegt der Vestibulärring nun parallel an einer Stelle des Weichgewebes von Lippen und Wangen an, die vergleichsweise wenig druckempfindlich ist.

Dass das Elastikband sich etwas mehr am Vestibulärring als am Lippenring erstreckt, liegt auch an deren Größenverhältnissen; typischerweise ist der Durchmesser des Lippenrings um beispielsweise 10 oder 20% größer als derjenige des Vestibulärrings.

Der Tragekomfort ist mit dem erfindungsgemäßen Folienspannelement wesentlich verbessert. Die Folie wird nach einwärts gezogen, so dass der Vestibulärring prominenter m Mund steht, so dass das Einsetzen des Folienspannelements erleichtert ist. Der Druck auf den Kieferknochen ist geringer, so dass Schmerzreaktionen vermindert werden, die in der Vergangenheit dazu geführt haben, dass der Vestibulärring aus dem Mund des Patienten entnommen worden und die anstehende Operation ohne Hilfsmittel fortgesetzt wurde.

Das erfindungsgemäße Elastikband ist bevorzugt schwimmend auf der Folie gelagert. Bevorzugt ist es mit einem, weiter bevorzugt mit zwei Ansätzen fixiert. Die Ansätze erstrecken sich von dem Vestibulärring entlang der Folie, aber so, dass das Elastikband auch an dieser Stelle von dem Vestibulärring beabstandet ist.

Das Elastikband ist so elastisch, dass es über einen Teil des Vestibulärrings stülpbar ist. Entweder ist ein kreisringförmiges Elastikband vorgesehen, oder es sind zwei Elastikbänder vorgesehen, die sich zu einem Kreis ergänzen. In diesem Fall ist jedes Elastikband über den zugehörigen Teil des Vestibulärrings stülpbar.

Zwar ist es möglich, das Elastikband an beliebigen Stellen an dem Folienelement zu befestigen. Die Befestigungen können beispielsweise durch zwei Punkte zwischen der Folie und dem Elastikband realisiert sein. Als besonders günstig hat es sich aber herausgestellt, zwei einander gegenüberliegende Ansätze vom Vestibulärring ausgehen zu lassen, und diese dort per Schweißpunkt auf der Folie zu befestigen, beispielsweise in einem Abstand von etwa 1cm von dem Vestibulärring.

In vorteilhafter Ausgestaltung ist es vorgesehen, dass der Ansatz an einer von dem Vestibulärring beabstandeten Verbindungsstelle mit dem Folienelement verbunden ist oder verbunden worden ist.

Es ist aber auch möglich, das Elastikband nur an einem Teilkreis der Folie anzubringen, die erfindungsgemäß erwünschte Wirkung stellt sich dann partiell ein.

In vorteilhafter Ausgestaltung ist es vorgesehen, dass das oder die Elastikbänder in dem über den Vestibulärring gestülpten Zustand an den von den Ansätzen weiter entfernt liegenden Verbindungsstellen das Folienelement weiter radial einwärts zieht als an den Ansätzen benachbart dem Vestibulärring.

In vorteilhafter Ausgestaltung ist es vorgesehen, dass das oder die Elastikbänder etwa (also +/- 30 %) die gleiche Stärke wie der Ansatz haben, dessen Breite aber das Doppelte bis das Zehnfache der Breite jedes Elastikbandes hat, insbesondere etwa das Dreifache.

In vorteilhafter Ausgestaltung ist es vorgesehen, dass je 2 Elastikbänder voneinander in tangentialer Richtung der Ringe um wenige mm beanstandet an dem gleichen Ansatz abstützen und von diesem ausgehend zum gegenüberliegenden Ansatz verlaufen.

In vorteilhafter Ausgestaltung der Erfindung ist es vorgesehen, dass die Elastikbänder sich in dem unumgestülpten Zustand biegeschlaff oder minimal gespannt, also mit deutlich weniger als 10 % Ihrer Maximalspannkraft, zwischen den Ansätzen erstrecken.

In weiterer vorteilhafter Ausgestaltung der Erfindung ist es vorgesehen, dass die Elastikbänder und die Ansätze zueinander einstückig sind und/oder aus dem gleichen Material bestehen.

Das Elastikband erstreckt sich bevorzugt mindestens zum großen Teil radial außerhalb der FolieEs ist bevorzugt über einen Teil des Vestibulärrings stülpbar und mit ihm ist die Folie radial einwärts spannbar. An mindestens einer, bevorzugt mindestens 2, Verbindungsstellen ist es mit der Folie und/oder dem Vestibulärring und/oder dem Lippenring verbunden.

Die Stärke und Breite des Elastikbandes läßt sich in weiten Bereichen an die Erfordernisse anpassen. Beispielsweise kann es eine kreisförmigen, einen ovalen, einen quadratischen oder einen rechteckigen oder einen beliebigen sonstigen Querschnitt aufweisen.

Bei einem flachen Querschnitt, also einem solchen, bei dem die Stärke geringer ist als die Breite, schmiegt es sich besonders gut an die Folie an.

Bevorzugt liegt es außen an die Folie an, und zwar insbesondere über deutlich mehr als die Hälfte der Ringerstreckung der Folie.

Sein Abstand zum Vestibulärring ist bevorzugt an der Stelle oder den Stellen, an denen es mit der Folie oder einem Ring verbunden ist, geringer, z. B. 1 mm bis 15 mm, und an hiervon beabstandeten Stellen, also z.B. an Stellen, die sich bei Postionierung am Mund seitlich erstrecken, größer, bis zum halben Abstand zwischen den Ringen, oder darüber hinaus.

Das Elastikband kann bei der Produktion über den Ring gestülpt werden und so ausgeliefert werden. Alternativ ist es auch möglich, den Kunden zu veranlassen, es über den Ring zu ziehen.

Es ist auch möglich, ein handelsübliches Gummiband, beispielsweise mit einem rechteckigen oder quadratischen Querschnitt, zu verwenden und dieses an mindestens einer Stelle, bevorzugt am Frenulum, mit der Folie und/oder einem Ring zu verbinden, und dieses sich ringsum um die Folie erstrecken zu lassen.

Das Elastikband verringert den Durchmesser der Folie an der Stelle, an der es sich erstreckt, und rafft diese.

In weiterer Ausgstaltung ist es vorgesehen, den Querschnitt und damit die Spannkraft des oder der Elastikbänder sich über deren Verlauf ändern zu lassen, und damit die Lippenanatomie zu simulieren:
Beispielsweise kann im seitlichen Bereich die Spannkraft höher eingestellt sein, und oben und unten geringer.

Die Folie wird dann seitlich stärker gerafft als oben und unten. Es ergibt sich für das zunächst kreisförmige Folienspannelement ein flachovaler Querschnitt, mind. Im Bereich der Folie.

Voraussetzung hierfür ist es, das Elastikband an mehreren Stellen, insbesondere auch seitlich, mit der Folie zu verbinden.

Es ist auch möglich, mehrere Elastikbänder in der Seitenansicht nebeneinander ringsum die Folie verlaufen zu lassen. Diese können die gleiche Elastizität und Spannkraft, aber bei Bedarf auch unterschiedliche Spannkräfte haben.

Beispielsweise kann ein mittleres Elastikband die größte Spannkraft, und sich zwischen diesem und je einem Ring erstreckende je eine geringere Spannkraft haben.

Bevorzugt wird die Folie automatisch in eine Lippenform oder in eine lippenähnliche Form vorgespannt, beispielsweise durch die vorstehend erläuterte Mehrfachanordnung von Elastikbändern.

Es ist auch möglich, ein Folienmaterial oder ein folienähnliches Material für die Ausbildung des oder der Elastikbänder zu verwenden. Ein solches Band kann eine Stärke zischen 0,05 und 1 mm und eine Breite zwischen 3 und 8 mm haben. Bevorzugt sind 0,3 mm * 5 mm.

Das Band kann auch auf die Folie kaschiert sein, so verläuft die Verbindung um den ganzen Umfang der Folie, und das Band ist sicher mit der Folie verbunden.

Es ist auch möglich, die Folie als geschichtete Folie mit mehreren Lagen auszubilden. Eine Lage kann dann das Elastikband bilden, bevorzugt ringförmig etwa in der Mitte zwischen den Ringen oder etwas zum Vestibulärring verlagert.

Das Elastikband kann auch als zentral zwischen den Ringen verlaufende Verdickung der Folie ausgebildet sein. Diese kann durchgängig sein oder Noppen aufweisen und erzeugt aufgrund ihrer größeren Spannkraft die erfindungsgemäße Einschnürung.

Das Profil der Folie in Queransicht betrachtet nähert sich durch das Elastikband und dessen Lage dem Lippenprofil an, was auch die Einführung des Folienspannelements in die Mundöffnung wesentlich erleichtert.

Ein besonderer Vorteil ergibt sich insofern daraus, dass die Einsetzbarkeit des Folienspannelements mit dem erfindungsgemäßen, eine Einschnürung der Folie bewirkenden Elastikband deutlich verbessert ist.

Erfindungsgemäß bevorzugt ist es, dass das Elastikband über einen Teil des Vestibulärrings stülpbar ist. Alternativ kann es auch über den Lippenring gestülpt werden, oder über einen Teil dieses Rings. Es ist bevorzugt an zwei Ansätzen mit dem betreffenden Ring verbunden, aber deutlich elastischer als dieser. Im entspannten Zustand ist es kürzer als der Umfang des Rings. Aufgrund seiner Elastizität lässt es sich aber ohne weiteres über dessen Außenumfang ziehen oder bewegen.

Für die Aktivierung des Folienbandes wird es kurzerhand, beispielsweise mit einem oder zwei Fingern, angehoben und über den betreffenden Teil des Vestibulärrings gezogen, also gestülpt. Dies kann auch werkseitig und maschinell unterstützt geschehen.

In vorteilhafter Ausgestaltung sind zwei Elastikbänder vorgesehen, die sich im entspannten Zustand wie biegeschlaffe Sehnen zwischen einander gegenüberliegenden Stellen des Vestibulärrings erstrecken. In dieser Form wird das Folienspannelement hergestellt.

Zwei Ansätze erstrecken sich von einander gegenüberliegenden Stellen des Vestibulärrings aufeinander zu, und jeder Ansatz mündet in zwei Enden eines Elastikbandes, an der Stützbasis.

Bevorzugt ist es vorgesehen, dass die Verbindungsstelle zwischen dem Vestibularring und der Stützbasis liegt.

Bevorzugt ist es vorgesehen, dass der Vorsprungrand des Vestibulärrings eine Polsterung aufweist, die an den tangentialen Verbindungsstellen der Ansätze ausgespart ist.

In vorteilhafter Ausgestaltung ist es vorgesehen, dass die Ansätze umgeschlagen werden und etwa 0,5cm bis 12mm von dem Vestibulärring entfernt an der Folie festgeheftet werden, beispielsweise durch Laserschweißen, Ultraschallschweißen,
Kleben oder Nähen oder eine anderes Fügeverfahren. Hierdurch wird zum einem das Elastikband oder die Elastikbänder unter Spannung gesetzt. Die Spannung in dem Elastikband ist dennoch je so gering, dass das betreffende Elastikband auf keinen Fall reißen kann.

Für die Aktivierung wird nun das rechte Elastikband über den rechten Teil des Vestibulärrings geschlagen oder gestülpt, und das linke Elastikband über den linken Teil geschlagen oder gestülpt.

Beide Elastikbänder zusammen bilden nunmehr einen Kreis, der von außen auf der Folie aufliegt und diese radial einwärts vorspannt. Die Vorspannung erfolgt in einem geringen Abstand von dem Vestibulärring, beispielsweise im Abstand von 1 cm, wobei der Abstand vom Vestibuläring mit zunehmenden Abstand von dem Ansatz zunimmt, so dass das Elastikband dort in Richtung Mitte zwischen Lippenring und Vestibulärring rutscht, in die energieärmste Stellung.

Das Folienelement, das biegeschlaff vormontiert worden war, wird durch das Elastikband an dieser Stelle radial einwärts vorgespannt. Zum anderen bildet es weiche Schlaufen, da bei dem nunmehr geringeren Durchmesser ein Materialüberschuss besteht.

Diese Schlaufen wirken übrigens dämpfend und sind auf der empfindlichen Lippenhaut ausgesprochen angenehm, da sie eine extreme Elastizität aufweisen.

In einer vorteilhaften Ausgestaltung der Erfindung ist es vorgesehen, dass die Folie an mindestens zwei einander gegenüberliegenden Stellen je einen Ansatz aufweist.

In einer weiteren vorteilhaften Ausgestaltung der Erfindung ist es vorgesehen, dass die Folie und die Elastikbänder einen gegenüber dem der Ringe um 80 bis 99% geringeren E-Modul aufweisen.

In einer weiteren vorteilhaften Ausgestaltung der Erfindung ist es vorgesehen, dass der Ansatz sich von dem Vestibulärring entlang des Folienelements wegerstreckt, und an seinem distalen Ende das oder die Elastikbänder führt und abstützt.

In einer weiteren vorteilhaften Ausgestaltung der Erfindung ist es vorgesehen, dass der Ansatz sich zwischen Lippenring und Vestibulärring über einen Bruchteil der Entfernung zwischen diesen erstreckt, insbesondere über weniger als 60% und besonders bevorzugt über 10 bis 45% und vorzugsweise über etwa 30%.

In einer weiteren vorteilhaften Ausgestaltung der Erfindung ist es vorgesehen, dass das der Ansatz etwa das Dreifache der Breite jedes Elastikbandes hat.

In einer weiteren vorteilhaften Ausgestaltung der Erfindung ist es vorgesehen, dass die Elastikbänder eine zerstörungsfreie Dehnfähigkeit von mindestens dem Doppeltem Ihrer Ausgangslänge haben, besonders bevorzugt das Zehnfache bis 50-fache.

In einer weiteren vorteilhaften Ausgestaltung der Erfindung ist es vorgesehen, dass die Elastikbänder und das Folienelement um 10 bis 30% dessen Durchmessers radial einwärts kontrahieren, und insbesondere Polsterfalten in dem Folienelement erzeugt.

In einer weiteren vorteilhaften Ausgestaltung der Erfindung ist es vorgesehen, dass ein Elastikband radial einwärts spannbar ist und an mindestens 2 Verbindungsstellen mit der Folie oder einem Ring verbunden ist oder verbindbar ist.

Gemäß einem weiteren Aspekt der Erfindung ist ein Folienspannelement für dentale Bearbeitungen, vorgesehen, mit dem Lippenring und dem Vestibulärring, zwischen denen und ggf. über welchen hinaus sich die Folie erstreckt. Die Folie und auch die Ringe sind elastisch verformbar. Die Folie ist an dem Vestibulärring oder dem Lippenring umgeschlagen. Hierbei ist die Folie mit beiden Ringen oder mit mindestens einem der beiden Ringe fest verbunden. Erst durch diese feste Verbindung kommt die Wirkung des erfindungsgemäßen Elastikbandes zum Tragen. Dieses erstreckt sich an dem von beiden Ringen beabstandeten und sich im Bereich zwischen diesen erstreckenden Ende der umgeschlagenen Folie entlang der Folie. Mit dem Elastikband ist die Folie an einer Stelle zwischen den Ringen radial einwärts spannbar. Hierdurch ist der Tragekomfort überraschend wesentlich verbessert.

In vorteilhafter Ausgestaltung dieser Lösung ist es vorgesehen dass das Elastikband als ringförmige Verdickung der Folie an deren Umschlagende ausgebildet ist und /oder dass das Elastikband im entspannten Zustand einen kleineren Durchmesser als die Ringe aufweist.

Gemäß einem weiteren Aspekt der Erfindung ist ein Folienspannelement für dentale Bearbeitungen, vorgesehen, mit dem Lippenring und dem Vestibulärring, zwischen denen und ggf. über welchen hinaus sich die Folie erstreckt, wobei die Folie und auch die Ringe elastisch verformbar sind. Die Folie ist mit beiden Ringen fest verbunden ist und von beiden Ringen beabstandet und sich zwischen diesen und im wesentliche parallel zu diesen erstreckend erstreckt sich mindestens ein Elastikband, insbesondere einstückig zu diesen, mit welchem die Folie an einer Stelle zwischen den Ringen radial einwärts spannbar ist.

In vorteilhafter Ausgestaltung dieser Lösung ist es vorgesehen, dass das Elastikband unter Realisierung von Noppen gebildet ist, die sich entlang des Elastikbandes, insbesondere in regelmäßigem Abstand zueinander, erstrecken.

Gemäß einem weiteren Aspekt der Erfindung ist ein Folienspannelement für dentale Bearbeitungen, vorgesehen, mit dem Lippenring und dem Vestibulärring, zwischen denen und ggf. über welchen hinaus sich die Folie erstreckt, wobei die Folie und auch die Ringe elastisch verformbar sind. Radial außerhalb der Folie erstreckt mindestens ein Elastikband erstreckt, das über einen Teil des Vestibulärrings und/oder des Lippenrings ziehbar ist und mit welchem die Folie radial einwärts spannbar ist. Das Elastikband ist an mindestens einer, bevorzugt mindestens 2, Verbindungsstellen mit der Folie oder einem Ring verbindbar oder verbunden.

Weitere Vorteile, Einzelheiten und Merkmale ergeben sich aus der nachfolgenden Beschreibung mehrerer Ausführungsbeispiele der Erfindung anhand der Zeichnung: Es zeigen:
- Fig. 1: eine perspektivische Ansicht auf ein erfindungsgemäßes Folienspannelement in einer Ausführungsform;
- Fig. 2: das Folienspannelement gemäß Fig. 1 in der Draufsicht;
- Fig. 3 und 3a: das Folienspannelement gemäß Fig. 1 und 2, jedoch mit über einen Teil des Vestibulärrings gestülptem Elastikband;
- Fig. 4: eine Detailvergößerung aus Fig. 3;
- Fig. 5: eine Detaildraufsicht auf Fig. 3;
- Fig. 6: einen Detailschnitt einer weiteren Ausführungsform des erfindungsgemäßen Folienspannelements;
- Fig. 7: eine Seitenansicht einer weiteren Ausführungsform des erfindungsgemäßen Folienspannelements;
- Fig. 8: eine Seitenansicht einer weiteren Ausführungsform des erfindungsgemäßen Folienspannelements;
- Fig. 9a: einen Schnitt durch eine weiteren Ausführungsform des erfindungsgemäßen Folienspannelements; und
- Fig. 9b: eine perspektische Ansicht einer weiteren Ausführungsform des erfindungsgemäßen Folienspannelements.

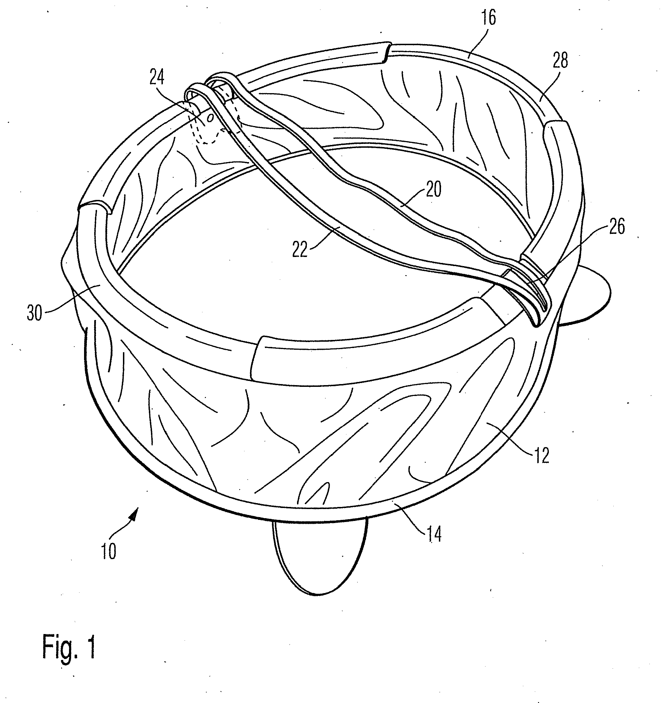

Das in Fig. 1 dargestellte Folienspannelement 10 weist eine Folie 12 auf, die sich zwischen ein Lippenring 14 und einem Vestibulärring 16 erstreckt.

Beide Ringe sind steif, aber dennoch elastisch verformbar.

Die Folie erstreckt sich gleichsam ringförmig oder mantelförmig, so dass der Innenraum sowohl des Vestibulärrings 16 als auch des Lippenrings 14 frei von Material ist.

Der Lippenring 14 ist bei diesem Ausführungsbeispiel in an sich bekannter Weise etwas größer als der Vestibulärring 16.

Quer über den Vestibulärring 16 erstreckt sich mindestens ein Elastikband 20. Im dargestellten Ausführungsbeispiel ist auch noch ein weiteres Elastikband 22 vorgesehen, das sich parallel zum Elastikband 20 erstreckt.

Die Bänder 20 und 22 können auf deutlich weiter voneinander beabstandet sein, so dass sich halbmondförmige Einschnürungen ergeben.

Die Verankerung beider Elastikbänder erfolgt an Ansätzen 24 und 26, wobei jedes Elastikband 20 und 22 sich zwischen diesen beiden Ansätzen 24 und 26 erstreckt.

Die Ansätze 24 und 26 sind ihrerseits an dem Vestibulärring 16 befestigt, und in dem dargestellten bevorzugten Ausführungsbeispiel zusätzlich an der Folie 12.

Die Elastikbänder 20 und 22 sind bevorzugt aus dem gleichen Material wie die Folie 12 und hochelastisch.

Sie sind so elastisch, dass es ohne weiteres möglich ist, sie über den betreffenden benachbarten Teil des Vestibulärrings zu stülpen, also das rechte Elastikband 20 über den rechten Teil 28 und das linke Elastikband 22 über den linken Teil 30 des Vestibulärrings 16.

Wenn die beiden Bänder 20 und 22 über den Vestibulärring 16 gestülpt sind, bilden sie im wesentlichen ein Kreis.

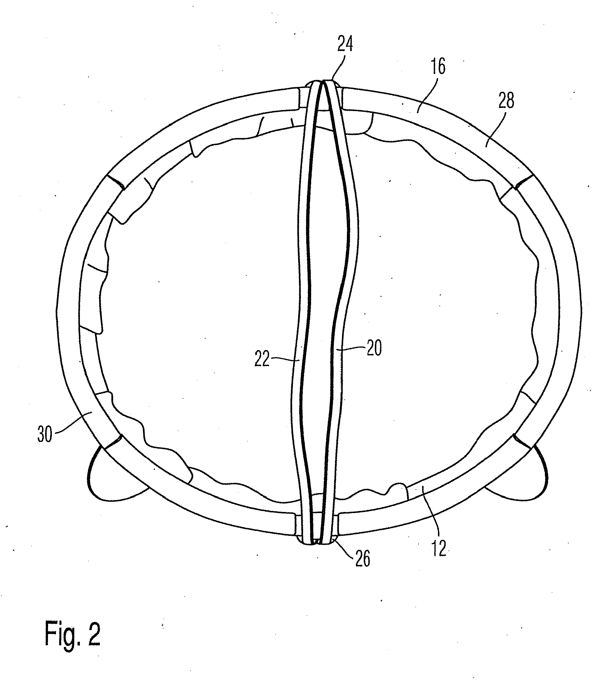

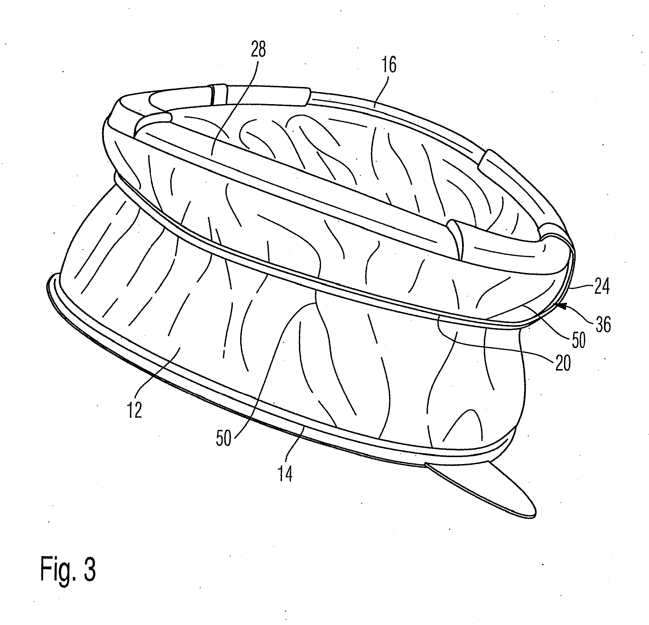

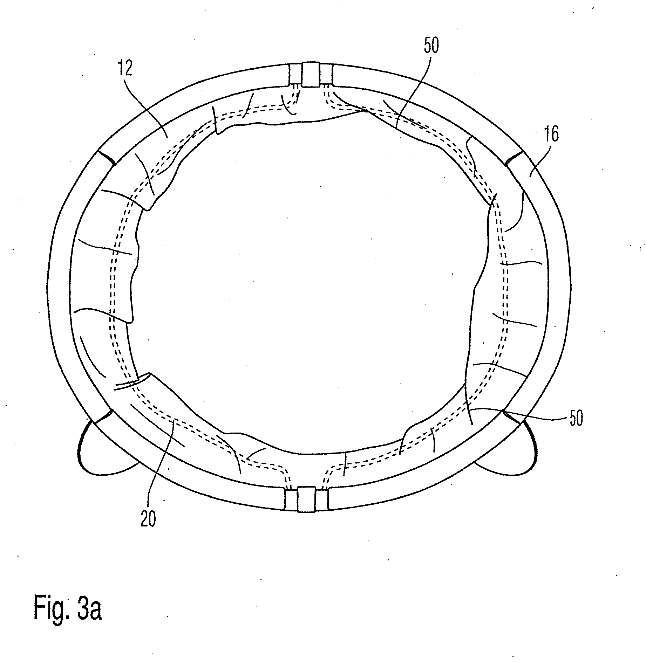

Aus Fig. 2 und 3 ist ersichtlich, in welcher Weise die Ansätze 24 und 26 ausgebildet sind. Jeder Ansatz ist wie eine Lasche an dem Vestibulärring befestigt, dergestalt, dass beide Ansätze 24 und 26 einander gegenüberliegen.

Jeder Ansatz erstreckt sich zudem radial außerhalb der Folie 12, über diese teilweise hinweg.

Der Ansatz 24 ist an der Stelle 36 per Ultraschallschweißen mit der Folie 12 verbunden, und der Ansatz 26 an der gegenüberliegenden Stelle .

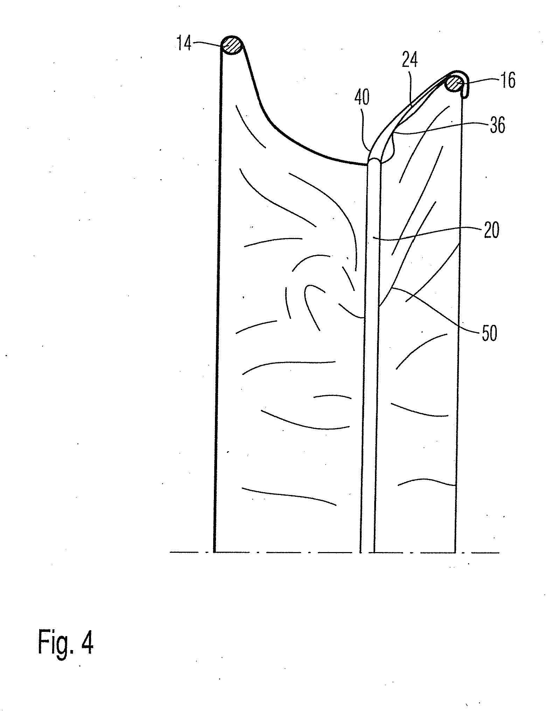

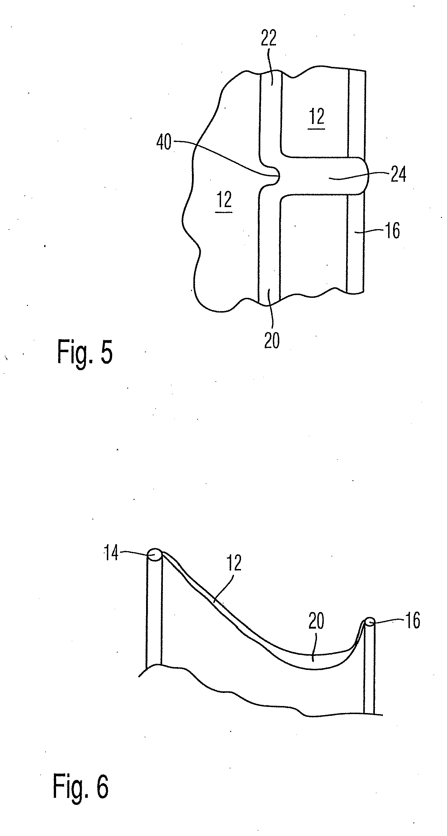

Diese beiden Stellen sind deutlich von dem Vestibulärring beabstandet. Der Ansatz 24 beziehungsweise 26 erstreckt sich jedoch über die je betrachtete Stelle 36 etwas hinaus, bis zu einer Stützbasis 40 (Fig. 4). Diese Stützbasis 40 ist an dem distalen Ende des Ansatzes 24 bzw. 26, und von dort gehen die Elastikbänder 20, 22 aus, und zwar voneinander divergierend (Fig. 5).

An dieser Stelle sind die Elastikbänder 20, 22 in der Position gemäß Fig. 2 umgeschlagen und erstrecken sich über den Vestibulärring 16 hinweg über das offene Ende des Folienspannelements 10.

Es ist jedoch möglich, beide Elastikbänder 20 und 22 zu spannen und diese über den Vestibulärring 16 zu ziehen beziehungsweise zu stülpen. Dieser Zustand ist in Fig. 3 dargestellt. In diesem Zustand ist das rechte Elastikband 20 weiter nach rechts gezogen, bis es sich über den rechten Teil 28 des Vestibulärrings 16 erstreckt. Etwas hinter diesem Teil wird es wieder losgelassen, so dass es sich erneut zusammenzieht und etwa die Form gemäß Fig. 3 annimmt.

In dieser Form spannt das Elastikband 20 die Folie 12 nach radial einwärts. Die Stelle, an der dies erfolgt, ist durch die Länge der Ansätze 24, 26 bestimmt, insbesondere durch die Lage der Verbindungsstellen 36.

Die Folie 20 wirft an dieser Stelle Schlaufen 50, die zusätzlich dämpfend wirken.

Das Elastikband 20 rutscht von selbst in die energieärmste Stellung, also diejenige, in der sich die Spannung zwischen Lippenring 14, Vestibulärring 16, Folie 12 und Elastikband 20 das Gleichgewicht hält.

Diese in Fig. 3 dargestellte Position ist optimal für den besten Tragekomfort des Patienten, wobei dennoch eine zuverlässige Abdeckung der Mundöffnung gegeben ist.

Aus dem Vergleich der Fig. 3a und 2 ergibt sich, dass die Foile 12 bei aktiviertem Elastikband 20 radial einen kleineren lichten Durchmesser hat und radial einwärts vorgespannt ist.

Aus Fig. 4 ist die aus Fig. 3 ersichtliche Position des Elastikbandes 20 im Detail ersichtlich. Die Stützbasis 40 ist etwas Richtung Lippenring 14 verlagert, von der Verbindungstelle 36, an der geschweißt ist, aus betrachtet.

Von dort geht das Elastikband 20 aus und schmiegt sich radial außen an die Folie 12 an.

Für eine exakte Position wird durch die Länge des Ansatzes 24, die den Abstand zum Vestibulärring 16 festlegt, bestimmt, und auch durch die Lage der Verbindungsstellen 36 Alternativ wäre es auch möglich, mit einem entsprechend längeren Ansatz die Führung des Elastikbandes 20 von dem Lippenring 14 ausgehend 24 realisieren.

Aus Fig. 5 ist der Ansatz 24 in der Draufsicht ersichtlich. Der Ansatz 24 besteht aus dem gleichen Material wie die Folie 12 und die Elastikbänder 20 und 22. Die Stützbasis 40 ermöglicht nahezu eine Kreisform des aus den Elastikbändern 20 und 22 gebildeten Ringes.

Es ist auch ersichtlich, dass der Ansatz 24 etwa die dreifache Breite der Elastikbänder 20 und 22 hat. Die Elastikbänder 20 und 22 sind bevorzugt von flach-ovalen Querschnitt, so dass sie sich gut an die Folie 12 anschmiegen können.

Fig. 6 zeigt eine weitere Ausführungsform der Erfindung. Bei dieser ist das erfindungsgemäße Elastikband 20 in die Folie 12 integriert. Es erstreckt sich insofern entlang der Folie ans ringförmige Verdickung dieser an ihrem gesamten Ringumfang.

Die Verdickung 20 hat eine größere Spannkraft als die Folie 12 im übrigen. Dies führt dazu dass die Folie an der Stelle der Verdickung 20 eine Einschnürung aufweist, die sich lippenfreundlich erstreckt, also an die gewölbte Rundform der Lippen angenähert ist.

Von außen und im Schnitt betrachtet bildet die Folie mit dem Elastikband 20 eine konkave Einwölbung aus, die die Lippe sanft einhüllt und zugleich dafür sorgt, dass der Vestibulärring vom Kieferknochen weggezogen wird.

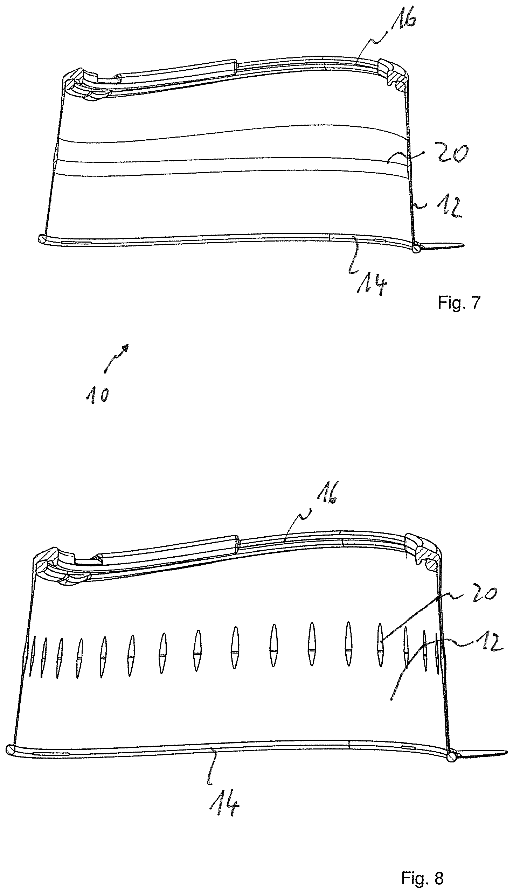

Bei der Ausführungsform gemäß Fig. 7 ist die Folie 12 zwischen dem Vestibulärring 16 und dem Lippenring 14 aufgespannt. Bei dieser Ausführungsform ist ein Elastikring 20 in die Folie 12 umlaufend eingearbeitet. Der Elastikring 20 erstreckt sich etwa im mittleren Bereich zwischen dem Vestibulärring 16 und dem Lippenring 14. Er ist gleichsam als Verdickung ausgebildet und hat bevorzugt einen rautenförmigen Querschnitt, wie es aus dem dargestellten Schnitt aus Fig. 7 ersichtlich ist.

Bei dieser Lösung zieht das Elastikband 20 die Folie 12 radial einwärts. Der radial nach außen wirkende Druck auf die Lippen wird so reduziert, so dass der Tragekomfort des Folienspannelements 10 verbessert ist.

Bei der Ausführungsform gemäß Fig. 8 ist das Elastikband 20 durch eine umlaufende Anordnung von Noppen 52 gebildet. Diese bewirken an dieser Stelle eine Materialstärkung, so dass dort wiederum eine Entlastung der Lippen durch radial einwärtigen Zug des Elastikbands 20 realisiert ist.

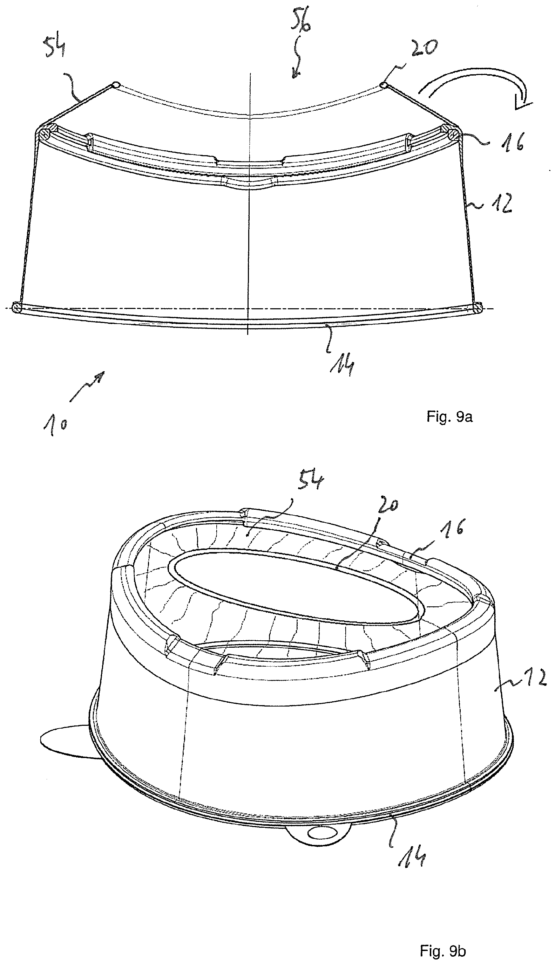

Bei der Ausführungsform gemäß Fig. 9a und Fig. 9b ist die Folie 12 über den Vestibulärring 16 hinaus verlängert. Sie läuft in einem Elastikband 20 aus, das ringförmig ausgebildet ist.

Die so erzeugte Folienverlängerung 54 lässt sich wiederum über den Vestibulärring 16 stülpen. Dann drückt das Folienband 20 am Ende der Folienverlängerung 54 den mittleren Bereich zwischen dem Vestibulärring 16 und dem Lippenring 14 radial nach einwärts, was wiederum zu der erwünschten Entlastung der Lippen führt und insofern den Tragekomfort verbessert.

Der Durchmesser der Öffnung 56 des Elastikbandes 20 lässt sich in weiten Bereichen an die Erfordernisse anpassen. Wenn das Elastikband 20 stärker ausgebildet ist, reduziert sich der Durchmesser der Öffnung 56 und damit auch der Durchmesser des mittleren Bereichs der Folie 12, nachdem die Folienverlängerung 54 einschließlich des Elastikbands 20 über den Vestibulärring 16 gestülpt ist.

Während hier das Elastikband 20 stülpbar ausgebildet ist, versteht es sich, dass es anstelle dessen auch möglich ist, das Elastikband 20 kurzerhand in den mittleren Bereich der Folie 12 zu ziehen.

## Patentansprüche

1. Folienspannelement für dentale Bearbeitungen, mit einem Lippenring (14) und einem Vestibulärring (16), zwischen denen sich eine Folie (12) erstreckt, wobei die Folie (12) und auch die Ringe elastisch verformbar sind, **dadurch gekennzeichnet, dass** sich radial außerhalb der Folie (12) mindestens ein Elastikband (20,22) erstreckt, das über einen Teil (28,30) des Vestibulärrings (16) und/oder des Lippenrings (14) ziehbar ist und mit welchem die Folie (12) radial einwärts spannbar ist und an mindestens einer Verbindungsstelle (36) mit der Folie (12) oder einem Ring (14,16) verbunden ist.

2. Folienspannelement nach Anspruch 1, **dadurch gekennzeichnet, dass** die Folie (12) und die Elastikbänder (20,22) elastischer als die Ringe sind und insbesondere einen gegenüber dem der Ringe um 80 bis 99 % geringeren E-Modul aufweisen.

3. Folienspannelement nach einem der vorhergehenden Ansprüche, **dadurch gekennzeichnet, dass** zwei Elastikbänder (20,22) vorgesehen sind, die so spannbar sind, dass sie sich zueinander zu einem Kreis ergänzen.

4. Folienspannelement nach einem der vorhergehenden Ansprüche, **dadurch gekennzeichnet, dass** jedes Elastikband (20,22) über einen Teil des Vestibulärrings (16) stülpbar oder ziehbar ist.

5. Folienspannelement nach einem der vorhergehenden Ansprüche, **dadurch gekennzeichnet, dass** der Ansatz (24) sich von dem Vestibulärring (16) wegerstreckt, insbesondere entlang des Folienelements (10), und an einer von dem Vestibulärring (16) beabstandeten Stützbasis (40), insbesondere an seinem distalen Ende, das oder die Elastikbänder (20,22) führt und abstützt.

6. Folienspannelement nach einem der vorhergehenden Ansprüche, **dadurch gekennzeichnet, dass** der Ansatz (24,26) bevorzugt einstückig an das Folienelement (10) angeformt ist, und/oder aus dem gleichen Material bestehen, und dass der Ansatz (24) sich insbesondere über einen Bruchteil der Entfernung zwischen Lippenring (14) und Vestibulärring (16) ersteckt, insbesondere über weniger als 60% und besonders bevorzugt über 10 bis 45 % und vorzugsweise über etwa 30 %.

7. Folienspannelement nach einem der vorhergehenden Ansprüche, **dadurch gekennzeichnet, dass** das Elastikband (20) einen flachen oder flachovalen Querschnitt aufweist, dessen Breite sich entlang des Folienelements (10) erstreckt und/oder das oder die Elastikbänder (20,22) in dem über den Vestibulärring (16) gestülpten Zustand sich entlang des Folienelements (10) außen an diesem erstreckt und dieses nach radial einwärts vorspannt.

8. Folienspannelement nach einem der vorhergehenden Ansprüche, **dadurch gekennzeichnet, dass** die Elastikbänder (20,22) eine zerstörungsfreie Dehnfähigkeit von mehr als dem 1,2-fachen, insbesondere mindestens dem Doppeltem Ihrer Ausgangslänge haben, besonders bevorzugt das Zehnfache bis 50-fache.

9. Folienspannelement nach einem der vorhergehenden Ansprüche, **dadurch gekennzeichnet, dass** sich von dem Vestibulärring (16) ausgehend ein Vorsprungrand in Verlängerung des Folienelements (10) von diesem weg wegerstreckt, und dass die Ansätze (24,26) an dem Vorsprungrand angebracht sind.

10. Folienspannelement nach einem der vorhergehenden Ansprüche, **dadurch gekennzeichnet, dass** die Elastikbänder (20,22) in dem über den Vestibulärring (16) gestülpten Zustand das Folienelement (10) radial einwärts kontrahiert, insbesondere um 10 bis 30 % dessen Durchmessers, und insbesondere Polsterfalten in dem Folienelement erzeugt.

11. Folienspannelement für dentale Bearbeitungen, mit einem Lippenring (14) und einem Vestibulärring (16), zwischen denen und ggf. über welchen hinaus sich eine Folie (12) erstreckt, wobei die Folie (12) und auch die Ringe elastisch verformbar sind, wobei die Folie (12) an dem Vestibulärring (16) oder dem Lippenring umgeschlagen ist, **dadurch gekennzeichnet, dass** die Folie mit beiden Ringen (14,16) fest verbunden ist und dass sich an dem von beiden Ringen (14,16) beabstandeten und sich im Bereich zwischen diesen erstreckenden Ende der umgeschlagenen Folie (12) entlang der Folie (12) mindestens ein Elastikband (20,22) erstreckt, mit welchem die Folie (12) an einer Stelle zwischen den Ringen (14,16) radial einwärts spannbar ist.

12. Folienspannelement nach Anspruch 11, **dadurch gekennzeichnet, dass** das Elastikband als ringförmige Verdickung der Folie an deren Umschlagende ausgebildet ist und /oder dass das Elastikband im entspannten Zustand einen kleineren Durchmesser als die Ringe aufweist.

13. Folienspannelement für dentale Bearbeitungen, mit einem Lippenring (14) und einem Vestibulärring (16), zwischen denen und ggf. über welchen hinaus sich eine Folie (12) erstreckt, wobei die Folie (12) und auch die Ringe elastisch verformbar sind, **dadurch gekennzeichnet, dass** die Folie mit beiden Ringen (14,16) fest verbunden ist und dass sich an dem von beiden Ringen (14,16) beabstandeten und sich zwischen diesen und im wesentliche parallel zu diesen erstreckend mindestens ein Elastikband (20,22) erstreckt, insbesondere einstückig zu diesen, mit welchem die Folie (12) an einer Stelle zwischen den Ringen (14,16) radial einwärts spannbar ist.

14. Folienspannelement nach Anspruch 13, **dadurch gekennzeichnet, dass** das Elastikband (20,22) unter Realisierung von Noppen gebildet ist, die sich entlang des Elastikbandes, insbesondere in regelmäßigem Abstand zueinander, erstrecken.

## Claims

1. A film clamping element for dental treatments, comprising a lip ring (14) and a vestibular ring (16), between which a film (12) extends, wherein said film (12) and also said rings are elastically deformable, **characterized in that** at least one elastic band (20, 22) extends radially to the outside of the film (12) which may be pulled over a part (28, 30) of the vestibular ring (16) and/or the lip ring (14) and with which the film (12) may be tensioned radially towards the inside and which is connected to the film (12) or a ring (14, 16) at at least one connection site (36).

2. The film clamping element as claimed in claim 1, **characterized in that** the film (12) and the elastic bands (20, 22) are more elastic than the rings and in particular have an E-modulus which is smaller compared to that of the rings by 80 to 99%.

3. The film clamping element as claimed in any of the preceding claims, **characterized in that** two elastic bands (20, 22) are provided which may be tensioned such that they complement one another to form a circle.

4. The film clamping element as claimed in any of the preceding claims, **characterized in that** each elastic band (20, 22) may be put over or pulled over a part of the vestibular ring (16).

5. The film clamping element as claimed in any of the preceding claims, **characterized in that** the attachment (24) extends away from the vestibular ring (16), in particular along the film element (10), and guides and supports the elastic band(s) (20, 22) at a support base (40) spaced apart from the vestibular ring (16), in particular at its distal end.

6. The film clamping element as claimed in any of the preceding claims, **characterized in that** the attachment (24, 26) is molded to the film element (10) preferably integrally, and/or consists of the same material, and that the attachment (24) extends in particular over a fractional part of the distance between the lip ring (14) and the vestibular ring (16), in particular over less than 60% and particularly preferably over 10 to 45% and more preferably over approximately 30%.

7. The film clamping element as claimed in any of the preceding claims, **characterized in that** the elastic band (20) comprises a flat or flat-oval cross-section whose width extends along the film element (10) and/or that in the state put over the vestibular ring (16), the elastic band(s) (20, 22) extend(s) along the film element (10) on its outside and pretension(s) it radially towards the inside.

8. The film clamping element as claimed in any of the preceding claims, **characterized in that** the elastic bands (20, 22) comprise a non-destructive elasticity of more than 1.2 times, in particular at least of twice their initial length, particularly preferably ten times to 50 times.

9. The film clamping element as claimed in any of the preceding claims, **characterized in that** a protrusion edge extends away from the film element (10) in extension thereof starting from the vestibular ring (16), and that the attachments (24, 26) are attached to the protrusion edge.

10. The film clamping element as claimed in any of the preceding claims, **characterized in that** in the state put over the vestibular ring (16) the elastic bands (20, 22) contract the film element (10) radially towards the inside, in particular by 10 to 30% of its diameter, and in particular produce padding folds in the film element.

11. A film clamping element for dental treatments, comprising a lip ring (14) and a vestibular ring (16), between which and possibly beyond which a film (12) extends, wherein said film (12) and also said rings are elastically deformable, wherein the film (12) is turned over at the vestibular ring (16) or the lip ring, **characterized in that** the film is fixedly connected with both rings (14, 16) and **in that** at least one elastic band (20, 22) extends at the end of the turned-over film (12) along the film (12) spaced apart from both rings (14, 16) and extending in the region therebetween, said elastic band being able to tension the film (12) radially towards the inside at a position between the rings (14, 16) .

12. The film clamping element as claimed in claim 11, **characterized in that** the elastic band is configured as a ring-shaped thickening of the film at its turned-over end and/or that the elastic band has a smaller diameter than the rings in its relaxed state.

13. A film clamping element for dental treatments, comprising a lip ring (14) and a vestibular ring (16), between which and possibly beyond which a film (12) extends, wherein said film (12) and also said rings are elastically deformable, **characterized in that** the film is fixedly connected with both rings (14, 16) and at least one elastic band (20, 22) extends at the end spaced apart from both rings (14, 16) and extending therebetween and substantially parallel thereto, in particular integrally therewith, said elastic band being able to tension the film (12) radially towards the inside at a position between the rings (14, 16).

14. The film clamping element as claimed in claim 13, **characterized in that** the elastic band (20, 22) is formed by realizing knobs which extend along the elastic band, in particular at regular distances to one another.

## Revendications

1. Élément de tension de feuille pour des traitements dentaires, avec un anneau labial (14) et un anneau vestibulaire (16), entre lesquels s'étend une feuille (12), où la feuille (12) ainsi que les anneaux sont déformables élastiquement, **caractérisé en ce qu'**au moins une bande élastique (20,22) s'étend radialement à l'extérieur de la feuille (12), qui peut être tirée sur une partie (28,30) de l'anneau vestibulaire (16) et/ou de l'anneau labial(14) et avec laquelle la feuille (12) peut être tendue radialement vers l'intérieur et reliée à la feuille (12) ou à un anneau (14,16) en au moins un point (36).

2. Élément de tension de feuille selon la revendication 1, **caractérisé en ce que** la feuille (12) et les bandes élastiques (20,22) sont plus élastiques que les anneaux et, en particulier, ont un module d'élasticité inférieur de 80 à 99 % à celui des anneaux.

3. Élément de tension de feuille selon l'une des revendications précédentes, **caractérisé en ce que** deux bandes élastiques (20,22) sont prévues, qui peuvent être tendues de manière à se compléter pour former un cercle.

4. Élément de tension en feuille selon l'une des revendications précédentes, **caractérisé en ce que** chaque bande élastique (20,22) peut être rabattue ou tirée sur une partie de l'anneau vestibulaire (16).

5. Élément de tension en feuille selon l'une des revendications précédentes, **caractérisé en ce que** l'appendice(24) s'étend à l'écart de l'anneau vestibulaire (16), en particulier le long de l'élément en feuille (10), et au niveau d'une base d'appui (40) se trouvant à distance de l'anneau vestibulaire (16), en particulier à son extrémité distale, qui guide et soutient la ou les bandes élastiques (20,22).

6. Élément de tension de feuille selon l'une des revendications précédentes, **caractérisé en ce que** l'appendice (24,26) est de préférence moulée en une seule pièce à l'élément en feuille (10), et/ou est faite du même matériau, et que l'appendice (24) s'étend en particulier sur une fraction de la distance entre l'anneau labial (14) et l'anneau vestibulaire (16), en particulier sur moins de 60 % et de manière particulièrement préférée sur entre 10 et 45 % et de préférence sur environ 30 %.

7. Élément de tension de feuille selon l'une des revendications précédentes, **caractérisé en ce que** la bande élastique (20) présente une section transversale plate ou ovale plate, dont la largeur s'étend le long de l'élément en feuille (10) et/ou la ou les bandes élastiques (20,22) à l'état retourné sur l'anneau vestibulaire (16) s'étendent le long de l'élément en feuille (10) à l'extérieur de ce dernier et les prétendent radialement vers l'intérieur.

8. Élément de tension de feuille selon l'une des revendications précédentes, **caractérisé en ce que** les bandes élastiques (20,22) ont une capacité d'allongement non destructive supérieure à 1,2 fois, en particulier au moins deux fois leur longueur initiale, de manière particulièrement préférée de dix à 50 fois.

9. Élément de tension de feuille selon l'une des revendications précédentes, **caractérisé en ce qu'**à partir de l'anneau vestibulaire (16), un bord saillant s'étend dans le prolongement de l'élément en feuille (10) en s'éloignant de celui-ci, et **en ce que** les appendices (24,26) sont montés sur le bord saillant.

10. Élément de tension de feuille selon l'une des revendications précédentes, **caractérisé en ce que** les bandes élastiques (20,22) à l'état retourné sur l'anneau vestibulaire (16) provoquent une contraction de l'élément en feuille (10) radialement vers l'intérieur, en particulier entre 10 et 30 % de son diamètre, et créent en particulier des plis de rembourrage dans l'élément en feuille.

11. Élément de tension de feuille, pour des traitements dentaires, avec un anneau labial (14) et un anneau vestibulaire (16), entre lesquels et éventuellement au-delà desquels s'étend une feuille (12), où la feuille (12) et également les anneaux sont déformables élastiquement, où la feuille (12) est rabattue sur l'anneau vestibulaire (16) ou sur l'anneau labial, **caractérisé en ce que** la feuille est reliée de manière fixe aux deux anneaux (14, 16) et **en ce qu'**à l'extrémité de la feuille (12) repliée, se trouvant à distance des deux anneaux (14, 16) et s'étendant dans la zone entre ceux-ci, s'étend le long de la feuille (12) au moins une bande élastique (20, 22) avec laquelle la feuille (12) peut être tendue radialement vers l'intérieur à un endroit situé entre les anneaux (14, 16).

12. Élément de tension de feuille selon la revendication 11, **caractérisé en ce que** la bande élastique est conçue comme un épaississement annulaire de la feuille à son extrémité de rabattement et/ou **en ce que** la bande élastique présente, à l'état détendu, un diamètre plus petit que les anneaux.

13. Élément de tension de feuille pour des traitements dentaires, avec une bague à lèvres (14) et une bague vestibulaire (16), entre lesquelles et éventuellement au-delà desquelles s'étend une feuille (12), où la feuille (12) et également les bagues sont élastiquement déformables, **caractérisé en ce que** la feuille est reliée aux deux bagues (14, 16) et **en ce qu'**au moins une bande élastique (20, 22) s'étend sur la partie qui est située à distance des deux anneaux (14, 16) et qui s'étend entre ceux-ci et sensiblement parallèle à ceux-ci, en particulier d'un seul tenant avec ceux-ci, avec laquelle la feuille (12) peut être tendue radialement vers l'intérieur en un point situé entre les anneaux (14, 16).

14. Élément de tension de feuille selon la revendication 13, **caractérisé en ce que** la bande élastique (20, 22) est formée en réalisant des plots qui s'étendent le long de la bande élastique, en particulier à une distance régulière les uns des autres.
